# EUROPÄISCHE PATENTSCHRIFT

(11) **EP 0 437 460 B1**
(45) Veröffentlichungstag und Bekanntmachung des Hinweises auf die Patenterteilung: **25.05.1994**
(21) Anmeldenummer: 89910858.3
(22) Anmeldetag: 26.09.1989
(51) Int. Cl.: C07H 15/04

(54) **VERFAHREN ZUR DIREKTEN HERSTELLUNG VON ALKYLGLYKOSIDEN**
PROCESS FOR DIRECTLY PRODUCING ALKYLGLYCOSIDES
PROCEDE DE PRODUCTION DIRECTE D'ALKYLGLUCOSIDES

(30) Priorität: 05.10.1988 DE 3833780
(43) Veröffentlichungstag der Anmeldung: 24.07.1991
(73) Patentinhaber: Henkel Kommanditgesellschaft auf Aktien, 40191 Düsseldorf (DE)
(72) Erfinder: HILL, Karlheinz, D-4006 Erkrath (DE); BIERMANN, Manfred, D-4330 Mülheim (DE); ROSSMAIER, Henry, D-4000 Düsseldorf 12 (DE); ESKUCHEN, Rainer, D-4000 Düsseldorf (DE); WÜST, Willi, D-4030 Ratingen (DE); WOLLMANN, Josef, D-5120 Herzogenrath 3 (DE); BRUNS, Andreas, D-4018 Langenfeld (DE); HELLMANN, Günter, D-4010 Hilden (DE); OTT, Karl-Heinz, D-4006 Erkrath (DE); WINKLE, Walter, D-4019 Monheim (DE); WOLLMANN, Klaus, D-5657 Haan (DE)
(86) Internationale Anmeldenummer: EP8901118
(87) Internationale Veröffentlichungsnummer: WO9003977

(56) Entgegenhaltungen:
- EP-A- 132 046
- EP-A- 165 721
- US-A- 3 839 318

## Beschreibung

Die Erfindung betrifft eine Weiterentwicklung des Verfahrens zur direkten Herstellung von oberflächenaktiven Alkylglykosiden, also den Acetalen aus Zuckern und aliphatischen Alkoholen, durch direkte säurekatalysierte Umsetzung der Alkohole mit den Zuckern unter Wasserabspaltung.

Der Begriff Alkylglykoside wird im folgenden für die Reaktionsprodukte aus Zuckern und aliphatischen Alkoholen verstanden, wobei als Zuckerkomponente die im folgenden als Glykosen bezeichneten Aldosen bzw. auch Ketosen in weitesten Sinne in Betracht kommen, wie z. B. die Glucose, Fructose, Mannose, Galactose, Talose, Gulose, Allose, Altrose, Idose, Arabinose, Xylose, Lyxose und Ribose. Vorzugsweise werden wegen der besseren Reaktionsfähigkeit die Aldosen verwendet. Unter den Aldosen kommt wegen ihrer leichten Zugänglichkeit und Verfügbarkeit in technischen Mengen insbesondere die Glucose in Betracht. Die nach dem Verfahren der Erfindung besonders bevorzugt hergestellten Alkylglykoside sind deshalb die Alkylglucoside. Der Begriff Alkyl in Alkylglykosid umfaßt im weitesten Sinne den Rest eines aliphatischen Alkohols beliebiger Kettonlänge, vorzugsweise eines primären aliphatischen Alkohols und insbesondere eines aus natürlichen Fetten erhältlichen Fettalkohols, so daß der Begriff gesättigte und ungesättigte Reste und deren Gemische einschließlich solcher mit verschiedenen Kettenlängen in Gemisch umfaßt. Die Begriffe Alkyloligoglykosid, Alkylpolyglykosid, Alkloligosaccharid und Alkylpolysaccharid beziehen sich auf solche alkylierten Glykosen, in denen ein Alkylrest in Form des Acetals an mehr als einen Glykoserest, also an einen Poly- oder Oligosaccharidrest gebunden ist. Diese Begriffe werden als untereinander gleichbedeutend angesehen. Dementsprechend ist ein Alkylmonoglykosid das Acetal eines Monosaccharids. Da man bei der säurekatalysierten Umsetzung von Zuckern und Fettalkoholen im allgemeinen Gemische erhält, werden im folgenden unter dem Begriff Alkylglykosid sowohl Alkylmonoglykoside als auch Alkylpoly(oligo)glykoside und insbesondere Mischungen daraus - einschließlich eventueller Nebenkomponenten - verstanden, sofern es nicht ausdrücklich auf die strukturellen Unterschiede ankommt.

Die oberflächenaktiven Alkylglykoside sind als Waschmittelrohstoffe bereits seit über 50 Jahren bekannt. So beschreibt die österreichische Patentschrift Nr. 135 333 die Herstellung von Laurylglucosid und Cetylglucosid aus Acetobromglucose und dem jeweiligen Fettalkohol in Gegenwart einer Base. Auch die Drektsynthese aus Glucose und Laurylalkohol mit Chlorwasserstoff als saurem Katalysator ist dort beschrieben. Nach der Lehre der deutschen Patentschrift 611 055 werden Alkylglucoside aus Pentaacetylglucose und dem Fettalkohol in Gegenwart von wasserfreiem Zinkchlorid hergestellt. Die Maltoside und Lactoside der aliphatischen Alkohole mit mehr als 8 Kohlenstoffatomen und ihre Verwendung als Tenside sind aus der deutschen Patentschrift Nr. 593 422 bekannt. Beispielsweise ist in dieser Veröffentlichung angegeben, daß Cetylmaltosid die Waschwirkung von Seife, die damals das wichtinste Tensid war, verbesser, was mit der Wirkung des Cetylmaltosids als Kalkseifendispergator erklärt wird. Aus den 60er und 70er Jahren stammen mehrere Vorschläge für die verbesserte Herstellung von Alkylglykosiden entweder durch direkte Umsetzung der Glykose, meist Glucose, mit einem Überschuß des Alkohols und einer Säure als Katalysator, oder unter Mitverwendung eines niederen Alkohols oder Glykols als Lösungsmittel und Reaktionspartner zu einem primären Reaktionsprodukt, aus dem durch Umacetalisierung mit dem längerkettigen Alkohol erst das oberflächenaktive Alkylglykosid gewonnen wird. In der US-Patentschrift 3,450,690 (Gibbons et al) wird ein Verfahren der Direktsynthese von Alkylglucosiden, allerdings mit C₁- bis C₈-Alkanolen, beschrieben, wobei Synthese-Nebenprodukte bzw. -Verunreinigungen, die im alkalischen Medium unerwünschte Verfärbungen hervorrufen, dadurch aus dem Rohprodukt entfernt werden, daß man dieses in wäßriger Lösung unter Erwärmen mit anorganischen oder organischen Basen wie z. B. Natriumhydroxid, Natriummethylat, Calciumhydroxid, Bariumhydroxid, Bariummethylat oder stark basischen Aminen behandelt. Dabei soll der saure Katalysator (z. B. Schwefelsäure) nicht nur neutralisiert werden, sondern es wird ein alkalischer pH-Wert von wenigstens 8 eingestellt. Nach dem Erhitzen auf Temperaturen zwischen 50 und 200 °C fallen die Verunreinigungen aus. Daraufhin wird abfiltriert und der Alkoholüberschuß abdestilliert. Als wäßrige Lösung wird in dieser Literaturstelle die Mischung aus dem Überschuß des alkoholischen Reaktionspartners und des bei der Reaktion entstandenen Wassers verstanden. In einigen Beispielen wird der überschüssige Alkohol (Ethanol) entfernt und zum Teil durch Wasser ersetzt. Nach dem Abfiltrieren des unlöslichen Niederschlages wird das Filtrat durch Behandeln mit Aktivkohle aufgehellt. Als äquivalente Maßnahme zur Aktivkohlebehandlung wird auch das Bleichen mit Wasserstoffperoxid erwähnt. Vorzugsweise wird als Base Calciumhydroxid verwendet. In der US-Patentschrift 3,839,318 (Mansfield et al) wird ein Verfahren der direkten Glucosidierung von langkettigen Alkoholen beschrieben, wobei die Reaktionsgeschwindigkeit durch Steuerung der Reaktionstemperatur und der Katalysatorkonzentration so geführt wird, daß das sich bildende Reaktionswasser aus dem Reaktionsgemisch möglichst schnell via azeotrope Destillation entfernt wird. Dabei wird vorzugsweise ein Kohlenwasserstoff als Lösungsmittel, z. B. Hexan oder Heptan, zugesetzt, um die rasche azeotrope Destillation des Wassers zu ermöglichen. Anschließend wird mit einer wäßrigen Lösung von Natriumhydroxid neutralisiert, wobei auch alkalische pH-Werte eingestellt werden können. Danach wird der überschüssige Alkohol auf die übliche Weise durch Destillation entfernt. Das Überführen des Reaktionsprodukts in eine wäßrige Paste und Bleichen dieser Paste mit Natriumperborat wird ebenfalls beschrieben.

Nach den Angaben der europäischen Patentanmeldung 132 046 (Procter & Gamble, Letton) wird bei einem Verfahren der Direktsynthese der saure Katalysator mit einer organischen Base neutralisiert, wobei ein enger pH-Wert-Bereich in der Nähe des Neutralpunkts (pH 6,6 bis 7, vorzugsweise 6,7 bis 6,8) eingestellt wird. Als organische Basen werden entweder die Alkali(Na, K, Li)- oder Erdalkali(Ba, Ca)- oder Aluminiumsalze von schwachen niedermolekularen Säuren, z. B. Natriumacetat, oder die entsprechenden Alkoholate, z. B. Natriumethylat, eingesetzt.

In der europäischen Patentanmeldung 96 917 (Procter & Gamble, Farris) wird ein verbessertes Verfahren der säurekatalysierten Direktsynthese beschrieben, wobei man ein Monosaccharid, vorzugsweise Glucose, kontinuierlich oder portionsweise so zu der Mischung aus Fettalkohol und Katalysator bei 80 bis 150 °C hinzufügt, daß nie mehr als 10 % nicht umgesetztes Monosaccharid im Reaktionsgemisch vorhanden sind.

Die europäische Patentanmeldung 77 167 (Rohm & Haas, Arnaudis) schlägt zur Verbesserung der Farbqualität von oberflächenaktiven Alkylglykosiden vor, daß man bei ihrer Herstellung einen üblichen sauren Katalysator zusammen mit einem saurem Reduktionsmittel aus der Gruppe der phosphorigen Säure, hypophosphorigen Säure, schwefeligen Säure, hyposchwefeligen Säure, salpetrigen Säure und/oder hyposalpetrigen Säure bzw. der entsprechenden Salze, verwendet.

Hellfarbige C₃- bis C₅-Alkylglucoside werden nach der Lehre der europäischen Patentanmeldung 102 558 (BASF, Lorenz et al) dadurch erhalten, daß man sie in Gegenwart eines sauren Katalysators und mindestens dazu äquivalenten Mengen eines Alkalmetallsalzes einer Borsäure, vorzugsweise Natriumperborat, herstellt.

Schließlich wird in der europäischen Patentanmeldung 165 721 (Staley, McDaniel et al) vorgeschlagen, die wäßrige Lösung eines oberflächenaktiven Alkylpolyglucosids zunächst mit einem Oxidetionsmittel, vorzugsweise mit einer Wasserstoffperoxidlösung, und anschließend mit einer Schwefeidioxidquelle, z. B. einer wäßrigen Lösung von Natriumbisulfit, zu behandeln. Die so erhaltenen Produkte sollen auch nach längerem Lagern farbstabil sein.

Bei der Herstellung von Tensidrohstoffen war man schon immer bestrebt, möglichst farblose Produkte zu erhalten. Farbige Verunreinigungen oder zunächst farblose Produkte, die sich beim Lagern verfärben, werden häufig als minderwertig oder unbrauchbar eingestuft, wenn mit ihnen keine ästhetisch befriedigenden Mischungen erzielt werden können. Bei der Weiterverarbeitung von Tensidrohstoffen spielt die Farbstabilität im alkalischen Medium eine besondere Rolle. Es ist zwar häufig möglich, technische Tensidrohstoffe durch Bleichen, beispielsweise mit wäßrigen Wasserstoffperoxidlösungen, in hellfarbige Produkte, die auch beim Lagern und im alkalischen Medium hellfarbig bleiben, überzuführen. Bei den oberflächenaktiven Alkylglykosiden, die bisher bekanntgeworden sind, versagt allerdings diese Bleichbehandlung; denn auch scheinbar aufgehellte Produkte nehmen wieder eine dunkelbraune Färbung an, wenn sie nach dem Bleichen mit wäßrigen Alkali bei erhöhter Temperatur behandelt werden. Den bekannten Herstellungsverfahren für Alkylglykoside, die auch eine verbesserte Farbqualität und Lagerstabilität anstreben, haftet der Nachteil an, daß man dabei entweder zusätzliche chemische Wirkstoffe während des Herstellungsverfahrens zusetzen muß, oder aber, daß ein solcher Zusatz für eine Nachbehandlung des Reaktionsproduktes erforderlich ist. Die vorliegende Erfindung setzt sich zum Ziel, ein neues Verfahren zur Herstellung von oberflächenaktiven Alkylglykosiden nach der sogenannten Direktsynthese bereitzustellen, wobei durch geeignete Wahl und Ausgestaltung der Verfahrensparameter dafür gesorgt wird, daß das im letzten Verfahrensschritt gebleichte Produkt beim Lagern und Weiterverarbeiten die erreichte Hellfarbigkeit auch unter alkalischen Bedingungen bei erhöhter Temperatur beibehält. Weiter ist es ein Ziel der Erfindung, die Verfahrensschritte so zu gestalten, daß man mit einem Minimum an chemischen Reaktionspartnern und einem Minimum an Verfahrensmaßnahmen auskommt. Schließlich ist es ein Ziel der Erfindung, die Verfahrensschritte so auszuwählen, daß eine Übertragung des Verfahrens in den großtechnischen Maßstab ohne scaling-up-Probleme möglich ist und sich das neue Verfahren für die Herstellung von oberflächenaktiven Alkylglykosiden in solchen Mengen eignet, daß das Verfahrensprodukt als Tensidrohstoff von der Waschmittelindustrie verarbeitet werden kann.

Es wurde nun gefunden, daß sich diese und weitere Ziele durch eine neuartige Kombination von sowohl an sich bekannten als auch neuen Verfahrensmerkmalen zu einem neuen Verfahren der Direktsynthese erreichen lassen.

Das erfindungsgemäße Verfahren betrifft die direkte Herstellung von Alkylglykosiden durch Acetalisierungsreaktion von höheren aliphatischen primären Alkoholen mit Glykosen, insbesondere mit Glucose, in Gegenwart eines sauren Katalysators, rascher Entfernung des Reaktionswassers, Neutralisation des Katalysators mit einer Base, Abdestillation des Alkoholüberschusses und Überführen des Reaktionsproduktes in eine wäßrige Paste und Bleichen dieser Paste, wobei der aliphatische Alkohol im molaren Überschuß zur Glykose eingesetzt wird, und wobei die Bildung und Abführung des Reaktionswassers unter Vakuum erfolgt und Reaktionstemperaturen über 80 °C angewendet werden. Das Verfahren ist dadurch gekennzeichnet, daß man:
a) Mischungen aus aliphatischem primärem Alkohol, Glykose und saurem Katalysator bei erhöhter Temperatur erzeugt und zur Reaktion bringt, wobei man entweder
   (i) eine Teilmenge des Alkohols mit dem Katalysator vorlegt, die Mischung erwärmt, und dann eine erwärmte Suspension der Glykose in der restlichen Alkoholmenge portionsweise oder kontinuierlich zu der Alkohol/Katalysatormischung zudosiert und dabei im Vakuum das entstehende Reaktionswasser abdestilliert, oder
   (ii) eine Mischung des gesamten Alkohols und der Glykose vorlegt, erwärmt und den sauren Katalysator zur erwärmten Mischung hinzugibt, dann ein Vakuum anlegt und weiter bis zum Einsetzen der Reaktion erwärmt und das entstehende Reaktionswasser abdestilliert,
b) dabei die Ansatzverhältnisse so wählt, daß das Molverhältnis Glykose zu aliphatischem Alkohol bei 1 : 2 bis 1 : 10, Vorzugsweise 1 : 3 bis 1 : 6 liegt,
c) das Reaktionsgemisch bei dieser Temperatur und diesem Unterdruck so lange hält, vorzugsweise unter Durchmischung, bis das Reaktionswasser vollständig entfernt ist
d) anschließend das Reaktionsgemisch auf ca. 90 ° abkühlt, dann eine organische oder anorganische basische Alkali-, Erdalkali- oder Aluminium- bzw. Alkali/Aluminiumverbindung in solchen Mengen hinzufügt, daß sich über die Neutralisation des sauren Katalysators hinaus ein pH-Wert von wenigstens 8, vorzugsweise 8 bis 10 einstellt, und vorzugsweise erst danach Normaldruck herstellt,
e) daß man vorzugsweise ohne vorherige Filtration den überschüssigen Alkohol aus dem alkalischen Gemisch im Vakuum auf an sich übliche, das Reaktionsprodukt schonende Weise auf einen Wert von unterhalb 5 Gew.-% des Reaktionsproduktes abdestilliert, und
f) daß man anschließend auf ca. 105 °C bis 130 °C abkühlt und durch Hinzugabe von Wasser eine 30- bis 70prozentige Paste erzeugt, die man durch vorzugsweise portionsweises Hinzufügen von Aktivsauerstoffverbindungen, vorzugsweise Wasserstoffperoxid, ca. 0,1 bis 5 Stunden bei ca. 80 °C rührt, wobei man gegebenenfalls durch Hinzufügen von Alkali, vorzugsweise Natronlauge, dafür sorgt, daß der pH-Wert während dieses Bleichprozesses bei 8 bis 10 bleibt.

Das Reaktionsprodukt wird in Form einer farblosen bis gelblichen wäßrigen Paste erhalten. Es wurde überraschenderweise gefunden, daß diese Paste beim Lagern und insbesondere auch beim Weiterverarbeiten im alkalischen Milieu die ursprüngliche Farbqualität nahezu unverändert beibehält. Die Farbstabilität des Produkts wird durch einen einfachen Test festgestellt. Dazu wird eine Probemenge des Produkts mit Wasser zu einer ca. 50%igen Paste vermischt und bei Normaltemperatur mit konzentrierter Natronlauge versetzt, so daß sich ein PH-Wert von ca. 12 bis 13 einstellt. Dann wird 30 Minuten lang auf 100 °C erhitzt. Bei den Ansätzen mit Verfahrensprodukten trat nach dieser Behandlung keine oder keine wesentliche Farbänderung ein. Die Farbzahlen für die Produkte wurden nach der Methode von KLETT bestimmt (5%ige Lösung in Wasser/Isopropylalkohol 1 : 1, 1 cm-Küvette, Blaufiter). Mit dieser Testmethode können Langzeitlagerversuche des pastenförmigen Produkts unter üblichen Bedingungen sowie Weiterverarbeitungsverfahren des gelagerten Produkts zu insbesondere Wasch- und Reinigungsmitteln und den dabei auftretenden alkalischen Bedingungen zuverlässig simuliert werden. Die Verfahrensprodukte besitzen vorzugsweise Klettzahlen von weniger als 35.

Als Glykose wird bei dem erfindungsgemäßen Verfahren vorzugsweise die Glucose verwendet. Handelsübliche Glucose enthält häufig 1 Mol Kristallwasser. Diese kristallwasserhaltige Glucose kann ohne weiteres verwendet werden. Dann sollte allerdings zweckmäßigerweise dieses Kristallwasser zusätzlich, und zwar vor dem Inkontaktbringen mit dem Katalysator aus dem Reaktionsmilieu durch thermische Maßnahmen entfernt werden. Nachdem aber auch wasserfreie Glucose in großen Mengen am Markt erhältlich ist, wird bevorzugt wasserfreie Glucose als feinteiliges Pulver engesetzt.

Als Katalysatoren sind generell alle sauren Verbindungen einschließlich der sogenannten Lewis-Säuren, welche die Acetalisierungsreaktion zwischen Fettalkohol und Zuckermolekül katalysieren, geeignet. Davon gelten die Schwefelsäure, Phosphorsäure, aliphatische und/oder aromatische Sulfonsäuren, vorzugsweise Paratoluolsulfonsäure und die sulfosauren Ionenaustauscherharze als besonders geeignet. Für das vorliegende Verfahren werden die Schwefelsäure und insbesondere die Paratoluolsulfonsäure, die eine geringere korrodierende Wirkung gegenüber Geräten und Leitungen aus Stahl hat, als Katalysator bevorzugt. Saure Ionenaustauscherharze sind im vorliegenden Fall ebenfalls geeignet, wenn die Abtrennung des Katalysators nach der Acetalisierung der Glykose vorgesehen ist. In einem solchen Falle wird vorzugsweise eine geeignete basische Verbindung nach der Abtrennung des sauren Austauscherharzes hinzugegeben, um das Gemisch auf pH 8 bis 10 einzustellen.

Als saure Katalysatoren können solche Verbindungen in solchen Mengen verwendet werden, daß deren resultierende Alkali-, Erdalkali- bzw. Aluminium-Salze im Produkt verbleiben können. Vorzugsweise wird eine Säure aus der Gruppe, bestehend aus Schwefelsäure, Phosphorsäure oder aliphatischen und/oder aromatischen Sulfonsäuren, vorzugsweise Paratoluolsulfonsäure, in einer Menge von vorzugsweise 0,005 bis 0,02 Mol pro Mol der eingesetzten Glykose verwendet.

Die Ansatzbedingungen für die drei Komponenten aliphatischer Alkohol, Glykose und Katalysator sind in weiten Grenzen variierbar. So ist es nach einer Variante des erfindungsgemäßen Verfahrens möglich, eine Mischung der Gesamtmengen aller Komponenten vorzulegen und durch Erwärmen die Reaktion einzuleiten. Nach einer anderen Variante wird eine Teilmenge des Alkohols mit dem Katalysator vorgelegt und die erwärmte Suspension der Glykose in der restlichen Alkoholmenge nach und nach hinzugefügt. Dabei gibt man im Falle von Laboransätzen einer portionsweisen und im Falle von großtechnischen Ansätzen einer kontinuierlichen Zugabe den Vorzug. Vorzugsweise werden bei der Dosierung die Zeitintervalle zwischen den Dosierportionen so gewählt, daß ständig eine im wesentlichen klare Phase vorliegt, d. h., daß die Menge der nicht umgesetzten Glykose im Reaktionsgemisch gering, d. h. bei nicht mehr als 10 %, gehalten wird. Auch das Ansatzverhältnis Glykose zu aliphatischem Alkohol kann in weiten Grenzen variiert werden. Auf diese Weise ist es möglich, den Verteilungsgrad zwischen Alkylmonoglykosid und Alkyloligoglykosiden im Reaktionsprodukt zu steuern.

Bei Laboransätzen und insbesondere bei den Ansätzen im großtechnischen Maßstab wurde gefunden, daß die Feindispergierung der Glykose im insbesondere langkettigen Alkohol einen wesentlichen positiven Einfluß auf die Qualität des Reaktionsproduktes hat. Diese Feindispergierung wird dadurch erreicht, daß man die feinpulverige Glykose, vor allem die Glucose, gegebenenfalls nach einer Feinmahlung, mit dem Alkohol intensiv vermischt. Für Laboransätze haben sich dafür die Verwendung eines hochtourigen üblichen Laborrührers oder aber eine Ultraschallbehandlung als geeignet erwiesen. Bei großtechnischen Ansätzen werden zur Feindispergierung mit Vorteil Inline-Mischer, beispielsweise ein Stator/Rotor-Mischer verwendet. Diese Feindispergierungsmaßnahme hat den erwünschten Nebeneffekt, daß sich dabei die Suspension erwärmt.

Während der Bildung und Abführung des Reaktionswassers wird ein Vakuum von etwa 10 bis 50 mbar angelegt. Während der Reaktion wird die Mischung erwärmt und vorzugsweise ständig durchmischt, was bei Laboransätzen durch einfaches Rühren geschieht, während bei großtechnischen Ansätzen dieses Durchmischen und Erwärmen durch Umpumpen über einen externen Flüssigkeitskreislauf mit einem Wärmeaustauscher erfolgt. Beim Zuführen der Wärmeenergie, die zur Aufrechterhaltung der Reaktionstemperatur benötigt wird, ist es wesentlich, daß zwischen Reaktorwand und Reaktionsgemisch nur eine geringe Temperaturdifferenz vorhanden ist, damit Überhitzungen vermieden werden. Um diese geringe Temperaturdifferenz einzustellen, genügt es bei Ansätzen im Labor, ein übliches Ölbad mit Thermostat zu verwenden und gleichzeitig das Reaktionsgemisch kräftig zu rühren. Bei Ansätzen im technischem Maßstab hat sich als besonders vorteilhaft erwiesen, die Wärmeenergie über einen externen Kreislauf, vorzugsweise bestehend aus einer Pumpe und einem Wärmeaustauscher, vorzunehmen. Zu diesem Zweck wird ständig ein Teil des Reaktionsgemisches über eine Rohrleitung abgezogen, im Wärmeaustauscher erwärmt und wieder in den Reaktor zurückgeführt. Auf diese Weise ist es möglich, hohe Reaktorwandtemperaturen, d. h. solche von mehr als 125 °C zu vermeiden und damit eine negative Auswirkung der Temperaturführung auf die Farbwerte des Endproduktes zu verhindern.

Die aliphatischen primären Alkohole, die erfindungsgemäß zur Umsetzung gebracht werden, können an sich beliebige Kettenlängen, d. h. solche von 1 bis etwa 30 Kohlenstoffatomen, aufweisen. Um oberflächenaktive Reaktionsprodukte, die als Tensidrohstoffe in Wasch- und Reinigungsmitteln eingesetzt werden können, zu erhalten, werden aliphatische primäre Alkohole mit 8 bis 20 Kohlenstoffatomen, insbesondere solche mit 12 bis 18 Kohlenstoffatomen, bevorzugt. Diese höheren aliphatischen Alkohole werden vorzugsweise aus technischen Fetten hergestellt. Selbstverständlich ist es aber auch möglich, synthetische primäre Alkohole wie z. B. die sogenannten Oxoalkohole nach dem erfindungsgemäßen Verfahren einzusetzen.

Arbeitet man nach der Dosierungsvariante des Verfahrens, dann legt man vorzugweise 30 bis 70 Gew.-% des Alkohols zusammen mit dem Katalysator vor, erwärmt die Mischung auf 100 bis 120 °C und gibt dann die Glykose als Suspension in der erwärmten restlichen Alkoholmenge hinzu, wobei man diese Zugabe vorzugsweise kontinuierlich unter Vakuum durchführt. Das entstehende Reaktionswasser wird ständig abdestilliert. Das Ende der Reaktion gilt dann als erreicht, wenn sich kein weiteres Reaktionswasser abscheidet. Um die Menge des Reaktionswassers zu bestimmen und so das Reaktionsende festzustellen, kann das Wasser beispielsweise durch Ausfrieren in einer Kühlfalle aufgefangen werden. Bei vorgegebenen Ansatzmengen und Reaktionsbedingungen kann somit die Reaktionszeit zuverlässig bestimmt werden, ohne daß jedes Mal das Auffangen und Messen des Reaktionswassers erforderlich wäre.

Bei der ebenfalls bevorzugten Verfahrensvariante, bei der man die Gesamtmenge des Ansatzes vorlegt, wird vorzugsweise so verfahren, daß man zunächst die Mischung aus Alkohol und Glykose vorlegt, diese Mischung unter Rühren erwärmt, d. h. bis ca. 80 °C Sumpftemperatur, und dann den sauren Katalysator zu der erwärmten Mischung hinzugibt. Danach wird ein Vakuum angelegt und weiter bis auf ca. 100 bis 120 °C erwärmt und das entstehende Reaktionswasser abdestilliert.

Da man, wie bereits ausgeführt, beim erfindungsgemäßen Verfahren die Alkohole mit einem weiten Kettenlängenbereich verwenden kann, läßt sich das Ausmaß des Vakuums auch so einstellen, daß dabei der Siedepunkt des Alkohols um wenigstens 30 °C gesenkt wird. Für die Umsetzung der langkettigen Fettalkohole mit C₁₂- bis C₁₈ Kohlenstoffatomen wird das Vakuum vorzugsweise auf einen Wert von 10 bis 50 mbar eingestellt.

Bei den als Alkoholkomponente besonders wichtigen höheren aliphatischen primären C₁₂- bis C₁₈-Alkoholen handelt es sich vorzugsweise um gesättigte und insbesondere um geradkettige Alkohole, wie sie durch die Hydrierung von nativen Fettsäuren im technischen Maßstab erhalten werden können. Typische Vertreter der höheren aliphatischen Alkohole, die nach dem erfindungsgemäßen Verfahren verwendet werden können, sind z. B. die Verbindungen n-Dodecylalkohol, n-Tetradecylalkohol, n-Hexadecylalkohol, n-Octadecylalkohol, n-Octylalkohol, n-Decylalkohol, Undecylalkohol, Tridecylalkohol. Da die Fettalkohole bevorzugt aus natürlichen Fettquellen stammen, kommen üblicherweise auch Gemische technischer Fettalkohole als Reaktionspartner in Betracht. Neben den eigentlichen Fettalkoholen sind auch verzweigtkettige primäre Alkohole, wie z. B. die sogenannten Oxoalkohole für die Umsetzung geeignet. Typische Oxoalkohole sind z. B. die Verbindungen C₁₂/C₁₃-Alkanol mit ca. 25 % hauptsächlich 2-Methylverzweigung Dobanol^{(R)} 23, und der entsprechende C₉-C₁₁-Alkanol Dobanol^{(R)} 91. Allerdings liegt ein Schwerpunkt des Verfahrens bei der Herstellung von Tensiden, die ausschließlich aus nachwachsenden Rohstoffen herstellbar sind.

Als basische Alkali-, Erdalkali- oder Aluminium- bzw. Alkali/Aluminiumverbindungen, die organisch oder anorganisch sein können, eignen sich beispielsweise Calciumhydroxid, Calciumoxid, Magnesiumhydroxid, Magnesiumoxid, die Zeolithe NaA oder NaX, vorzugsweise in Kombination mit Calciumhydroxid, wasserfreies Natriumcarbonat, Kaliumcarbonat, Magnesium- und Calciumcarbonat, Natriummethylat, Natriumethylat, Magnesiummethylat, Magnesiumethylat, Natrium- bzw. Magnesiumpropylat oder -butylat, d. h. die Alkoholate von niedrigsiedenden Alkoholen, vorzugsweise C₁-C₄-Alkoholen. Als besonders bevorzugte anorganische basische Verbindung kommt Magnesiumoxid in Betracht, während als besonders bevorzugte organische Base ein Magnesiumalkoholat, insbesondere das Ethanolat des Magnesiums eingesetzt wird. Dabei können sowohl das Magnesiumoxid als auch das Magnesiumalkoholat partiell, d. h. bis zur Hälfte des Mol-Gewichts durch gepulvertes Natriumhydroxid in äquivalenten Mengen ersetzt werden.

Es gilt als ein besonderes Merkmal des Verfahrens, daß man die Zusätze der basischen Verbindungen so steuert, daß über eine Neutralisation des sauren Katalysators hinaus ein Überschuß der basischen Verbindung vorliegt, so daß das Reaktionsgemisch deutlich basisch reagiert und deshalb vorzugsweise einen pH-Wert zwischen 8 und 10 aufweist. Der pH-Wert wird in einer 10%igen wäßrig/alkoholischen Emulsion einer Probemenge mit einem üblichen pH-Meßgerät gemessen.

Die Abdestillation des Alkoholüberschusses erfolgt auf eine das Reaktionsprodukt schonende Weise durch die geeignete Wahl von Vakuumdestillations-Methoden. Dabei wird bei einem Vakuum im Bereich von 0,01 bis 1 mbar destilliert. An sich gehört zur produktschonenden Destillation auch das Einstellen einer möglichst niederen Sumpftemperatur, worunter man die Temperatur des siedenden Gemisches versteht. Es hat sich jedoch im vorliegenden Fall überraschenderweise als vorteilhaft gezeigt, die Reaktionsmischung bis auf eine Sumpftemperatur von 160 bis 180 °C, insbesondere von 160 bis 170 °C, zu erhitzen; und zwar unabhängig davon, ob ein derartig hoher Wert bei der vorgegebenen Vakuumleistung zur Abdestillation des überschüssigen Alkohols notwendig wäre. Zwar führt eine derart hohe Sumpftemperatur zunächst unmittelbar zu einem Rohprodukt mit verschlechterter Farbqualität. Es konnte jedoch in unerwarteter Weise gezeigt werden, daß gerade die bei der hohen Sumpftemperatur behandelten Produkte nach dem Bleichen eine hellere Farbe und eine bessere Alkalistabilität im Sinne des oben angegebenen Tests besitzen als solche Produkte, die bei niederen Sumpftemperaturen behandelt worden waren und vor dem Bleichen eine scheinbar bessere Farbqualität aufwiesen. Es ist infolgedessen ein weiteres wichtiges Merkmal des Verfahrens, die Reaktionsmischung während der Verfahrensstufe der Entfernung des Alkoholüberschusses im Hochvakuum auf die hohe Sumpftemperatur von ca. 160 bis 180 °C zu bringen, selbst wenn diese hohe Sumpftemperatur nicht für die Abdestillation des Alkoholüberschusses erforderlich wäre, was bei den kürzerkettigen Fettalkoholen der Fall ist.

Für die Destillation von Laboransätzen werden zur Entfernung des Alkoholüberschusses übliche Vakuumdestillationsgeräte benutzt. Bei technischen Ansätzen im Produktionsmaßstab wird die Abdestillation des Alkohols vorzugsweise nach einem zweistufigen Verfahren durchgeführt, wenn es sich bei dem Fettalkohol um einen solchen aus dem Kohlenstoffkettenlängenbereich 12 bis 20 handelt, wobei man in einer ersten Stufe eine Abreicherung des Fettalkoholanteils auf Werte von ca. 40 bis ca. 20 % mit einem Dünnschichtverdampfer oder einem Fallfilmverdampfer durchführt, und wobei diese erste Stufe auch zur Entgasung des Reaktionsgemisches dient. In einer zweiten Stufe wird mit einem Kurzwegverdampfer bzw. einem Dünnschichtverdampfer die weitere Fettalkoholabreicherung auf den gewünschten Endwert eingestellt. Dieser Endwert kann, bezogen auf das Endprodukt, bei Werten unterhalb 0,5 Gew.-% liegen, wenn das Produkt praktisch frei von dem Fettalkohol sein soll. Für den Fall, daß gezielt Fettalkoholanteile im Endprodukt erwünscht sind, können diese Fettalkoholanteile bei 3 bis 5 Gew.-% eingestellt werden. Es hat sich gezeigt,
daß Verfahrensendprodukte mit einem Fettalkoholanteil von über 2 Gew.-%, vorzugsweise 3 bis 5 Gew.-%, gewisse anwendungstechnische Vorteile besitzen.

Für die schonende Auftrennung von temperaturempfindlichen Substanzgemischen gilt generell, daß sich zur schonenden Verdampfung bei reduziertem Druck Fallfilmverdampfer und insbesondere Dünnschichtverdampfer besonders gut eignen, weil sich in diesen Geräten extrem kurze Verweilzeiten bei den erforderlichen höheren Temperaturen erreichen lassen. In dem vorliegenden Fall eignen sich zur Abreicherung des überschüssigen Fettalkohols mit 10 bis 18 Kohlenstoffatomen vom Alkylglykosid mit besonders guten Tensideigenschaften vor allem Dünnschichtverdampfer. Als Dünnschichtverdampfer bezeichnet man solche Verdampfer, in denen ein hochviskoses schwer siedendes Gemisch auf eine beheizte Wand aufgegeben und dort durch rotierende Wischelemente mechanisch verteilt wird. Dabei werden dünne Flüssigkeitsschichten bzw. Flüssigkeitsfilme erzeugt, und die Filmoberflächen werden ständig erneuert. Die entstehenden Dämpfe strömen entgegen dem Fluß des Produktfilms und verlassen den Verdampfer in den außen angeordneten Kondensator. Im Dünnschichtverdampfer wird im allgemeinen bei Drucken von nur einigen mbar gearbeitet und die Verweildauer für das Produkt beträgt nur wenige Sekunden. In einer zweistufigen Anlage, wie sie in dem erfindungsgemäßen Verfahren bevorzugt benutzt wird, fungiert das erste Verdampfergerät auch als Vorentgaserstufe für das in zweiter Stufe benutzte Verdampfergerät. Gase, die in der viskosen Flüssigkeit gelöst sind, werden so im Zuge der Abreicherung des Reaktionsproduktes an überschüssigem Fettalkohol im ersten Dünnschichtverdampfer aus der Flüssigkeit entfernt. Bei dem als zweites Verdampfergerät auch bevorzugt eingesetzten Kurzwegverdampfer handelt es sich im Prinzip um einen Wischfilmverdampfer mit einem im Verdampfer eingebauten Kondensator. Diese Geräte eignen sich zur Destillation hochsiedender und temperaturempfindlicher Produkte im Bereich 10⁻¹ bis 10⁻⁴ mbar. Ähnlich wie bei dem Dünnschichtverdampfer wird auch bei dem Kurzwegverdampfer die Flüssigkeit mechanisch auf der Heizfläche durch Wischer verteilt. Erfindungsgemäß wird im Kurzwegverdampfer bzw. Dünnschichtverdampfer als zweiter Stufe der überschüssige Alkohol auf praktisch beliebige Restgehalte, die unter 1 % liegen können, entfernt. Die Zweistufenanordnung der Verdampfergeräte gestattet hohe Durchsätze in Verbindung mit der gezielten Einstellung des erwünschten Restgehaltes an Fettalkohol im Endprodukt. Für technische Zwecke lassen sich Dünnschicht- und Kurzwegverdampfer so dimensionieren, daß Durchsätze von bis zu 300 kg/qm und Stunde ohne weiteres möglich sind. Die erfindungsgemäß bevorzugte Verfahrensvariante mit der zweistufigen Fettalkoholabreicherungsanlage läßt sich prinzipiell auch in der passenden Dimensionierung für die Aufarbeitung von Laboransätzen verwenden.

Die erfindungsgemäß hergestellten Alkylglykoside stellen Gemische dar, die im wesentlichen aus Alkylmonoglykosid und den Alkyloligoglykosiden, hier im wesentlichen beschränkt auf Di- und Triglykoside, und geringen Anteilen an Tetra- und Pentaglykosiden, bestehen. Die Verteilung zwischen Mono- und Oligoglykosiden in dem Verfahrensprodukt ergibt einen rechnerischen Oligomerisierungsgrad, der zwischen 1 und 5 liegt. Vorzugsweise wird das Verfahren so geführt, daß der Oligomerisierungsgrad zwischen 1 und 1,5 liegt, wobei die Menge an Alkylmonoglykosid, bezogen auf die Gesamtmenge, aus Alkylmonoglykosid und Alkyloligoglykosid deutlich über 70 Gew.-% liegt. (Zur Definition des Oligomerisierungsgrades siehe Paul J. Flory, Principles of Polymor Chemistry, Cornell University Press, Ithaca, New York, 1953, Seiten 35 bis 37). Die Gesamtmenge der übrigen Nebenbestandteile liegt meist unter 20 Gew.-%. Von diesen Nebenbestandteilen ist der Fettalkoholanteil variabel, da er von der Intensität der Fettalkoholabdestillation abhängt. Die Restalkoholmenge ist in den Verfahrensprodukten auf einen bevorzugten Bereich von 0,2 bis 5 Gew.%, insbesondere 0,5 bis 2,5 Gew.-%, eingestellt. Die Restmengen an nicht umgesetzter Glykose liegen unterhalb von 1 %. Die Anteile an polymerer Glucose im Verfahrensprodukt liegen bei 2 bis 20, vorzugsweise 5 bis 20 Gew.-%. Die Mengen der Neutralisationsprodukte aus saurem Katalysator und basischer Verbindung und eventuellem Überschuß an dieser basischen Verbindung im Verfahrensprodukt liegen bei 0,5 bis 1,5 Gew.-%.

Diese Mengenangaben beziehen sich auf das Reaktionsprodukt, wie es unmittelbar nach der Abdestillation des Fettalkoholüberschusses vorliegt. Das eigentliche Verfahrensprodukt ist die daraus durch Behandeln mit warmem Wasser und Bleichen mit Aktivsauerstoffverbindungen, insbesondere Wasserstoffperoxid resultierende wäßrige Paste mit 30 bis 60 Gew.-% Wasseranteil. Die Menge der Aktivsauerstoff-Verbindung beträgt dabei im allgemeinen 0,2 bis 1,5 Gew.-%, berechnet als H₂O₂ und bezogen auf die Menge des Produkts nach der Alkoholabtrennung. Da bei dem Bleichvorgang der pH-Wert abnimmt, wird zusammen mit der Perverbindung eine Base, z. B. Natriumhydroxid, zugesetzt, um pH-Werte zwischen 8 und 10 aufrechtzuerhalten. Die resultierende Lösung bzw. Paste enthält vorzugsweise die aus der Neutralisation des Katalysators und dem Bleichvorgang stammenden, nicht abgetrennten Salze. Es hat sich gezeigt, daß auf vielen Anwendungsgebieten diese nicht abgetrennten Salze in der wäßrigen Alkylglykosid-Paste nach Menge und Art nicht stören. Vielmehr handelt es sich hier um Verbindungen, die ohnehin übliche Bestandteile üblicher Wasch- und Reinigungsmittel darstellen. Das pastenförmige Verfahrensprodukt wird im allgemeinen, was seinen pH-Wert betrifft, so belassen, wie es nach dem Bleichprozeß anfällt, d. h. die Paste besitzt einen pH-Wert zwischen 8 und 10. Für besondere Anwendungszwecke kann der pH-Wert durch Zugabe einer sauren Verbindung, deren Anwesenheit für die weitere Verwendung günstig zumindest aber unschädlich ist, auf Werte um den Neutralpunkt herabgesetzt werden. Geeignete saure Zusätze sind beispielsweise saure Salze, wie Natrium- oder Kaliumhydrogensulfat, anorganische Säuren wie Schwefelsäure oder organische Säuren wie Citronensäure oder Sulfonat- bzw. Sulfattenside in der Säureform.

Für eine längere Lagerzeit bzw. einen längeren Transport des pastenförmigen Reaktionsprodukts kann es von Bedeutung sein, daß mikrobielle Abbauprozesse wirksam verhindert werden. Zweckmäßigerweise enthält deshalb das erfindungsgemäß hergestellte pastenförmige Reaktionsprodukt einen die Lagerungsbeständigkeit verbessernden üblichen antimikrobiellen Zusatz in üblicher Menge. Dieser Zusatz besteht beispielsweise aus 0,1 bis 0,2 Gew.-% Glutardialdehyd.

Eine besonders bevorzugte Ausführungsform des Verfahrens zur Herstellung von hellfarbigen und farbstabilen Alkylglykosiden nach der Methode der Direktsynthese ist durch die Anwendung der folgenden kumulativen Verfahrensschritte gekennzeichnet:
1. Die Glykose, insbesondere die Glucose, wird im Alkohol mit hochtourigen Rührern oder mit anderen hochwirksamen technischen Mischvorrichtungen feindispergiert.
2. Die zur Neutralisation des Säurekatalysators, vorzugsweise einer Sulfonsäure, verwendete Base besteht ganz oder überwiegend aus Magnesiumoxid.
3. Die Basenmenge wird so berechnet, daß über die eigentliche Neutralisation hinaus eine basisch reagierende Mischung, vorzugsweise von pH 8 bis 10 erhalten wird.
4. Das Reaktionsgemisch wird nach der Neutralisation nicht filtriert.
5. Beim Abdestillieren des überschüssigen Alkohols im Vakuum wird schließlich auf eine Sumpftemperatur von 160 bis 180 °C erhitzt bzw. die Beheizungstemperatur im Verdampfergerat der zweiten Stufe auf etwa 170 °C bis 180 °C gebracht.

Die hohe Produktqualität des Endprodukts nach der Bleiche ist auf die kumulative Anwendung dieser Verfahrensschritte zusammen mit den anderen Verfahrensschritten zurückzuführen. Sinngemäß läßt sich diese Kombination von Verfahrensparametern auch bei den anderen Herstellungsverfahren für Alkylglykoside wie z. B. beim Umacetalisierungsverfahrens mit Butanol oder Propylenglykol, bzw. bei solchen Verfahren, die Polyglykosen, insbesondere Stärke und Stärkeabbauprodukte als Ausgangsstoffe benutzen, anwenden.

### Beispiele

Die folgenden Beispiele beschreiben das erfindungsgemäße Verfahren.

### Beispiel 1:

### In diesem Beispiel wird das erfindungsgemäße Verfahren der Direktsynthese von C₁₂-Alkylglucosid im Labormaßstab nach der Methode der portionsweisen Zugabe einer Glucose/Fettalkoholsuspension (Slurry-Variante) beschrieben.

In einem 2-Liter-Dreihalskolben mit Rührer, Tropftrichter und Kolonne zur Wasserabscheidung wurden 559 g (3 Mol) n-Dodecanol und 2,2 g (11,2 mMol) Paratoluolsulfonsäure vorgelegt und auf 110 bis 114 °C erhitzt. Dann wurde eine Suspension von 180 g (1 Mol) wasserfreier Glucose (Puridex der Fa. Cerestar Deutschland GmbH) in weiteren 559 g (3 Mol) n-Dodecanol portionsweise, und zwar in 10 Portionen, in Abständen von 5 Minuten hinzudosiert. Dabei wurde vor der ersten Dosierung er Vakuum, das zwischen 10 und 15 mbar schwankte, angelegt. Die Glucose/Fettalkoholsuspension war vor dem Dosieren ebenfalls auf ca. 110 °C erwärmt worden. Das Reaktionswasser wurde über den Destillationsaufsatz aus dem Reaktionsmilieu entfernt und in einer Kühlfalle, die mit flüssigem Stickstoff gekühlt war, ausgefroren und aufgefangen. Es wurden insgesamt 19 g Wasser gemessen.

Im Anschluß daran wurde noch 120 Minuten ebenfalls bei 110 bis 115 °C nachgerührt. Das Reaktionsgemisch wurde dann auf 90 °C abgekühlt und bei Normaldruck mit 2,0 g (17,5 mMol) Magnesiumethylat versetzt. Die Mischung wurde 30 Minuten lang gerührt. Das Reaktionsgemisch hatte anschließend einen pH-Wert zwischen 9 und 10. Bei einem Vakuum von 0,1 bis 0,01 mbar und einer Sumpftemperatur von 120 bis 170 °C wurde der überschüssige Alkohol aus dem Reaktionskolben abdestilliert. Die Destillatmenge betrug 976 g; der Destillationsrückstand, d. h. das eigentliche Produkt fiel in einer Menge von 299,1 g an. Dieser Rückstand wurde bei einer Temperatur von 90 °C mit Wasser und 4,5 g einer 35%igen H₂O₂-Lösung versetzt und so unter Rühren innerhalb von 120 Minuten zu einer 60%igen Paste verarbeitet. Während des Bleichprozesses wurde der pH-Wert kontrolliert und durch Hinzufügen von NaOH 50%ig auf dem Wert von pH 9 gehalten.

Produktkenndaten: Hydroxylzahl: 656; restlicher Fettalkohol: 0,7 Gew.-%; Dodecylmonoglucosid: 67 Gew.-%; Dodecyldiglucosid: 16 Gew.-%; Dodecyltriglucosid: 5 %; Dodecyltetraglucosid 2 Gew.-%; Dodecylpentaglucosid 1 Gew.-%; Polyglucose: 7 Gew.-%, Glucose unter 1 Gew.-%. Klettzahlen: nach der Bleiche: 20; nach dem Farbstabilitätstest: 25.

### Beispiel 2:

### Dieses Beispiel beschreibt die Herstellung eines C₁₂/C₁₄-Alkylglucosids aus wasserfreier Glucose und einem technischen Fettalkohol (Gemisch aus ca. 75 Gew.-% Dodecanol und ca. 25 Gew.-% Tetradecanol) nach der sogenannten Batch-Variante (Ansatz mit der Gesamtmenge dem Reaktionskomponenten) im Kleintechnikumsmaßstab

In einem 150 Liter-Kessel aus Edelstahl wurden 25,0 kg (129 Mol) des Dodecanol/Tetradecanol-Gemisches (Lorol S, Henkel KGaA) und 7,7 kg (43 Mol) wasserfreie Glucose (Puridex) vorgelegt und auf etwa 80 °C unter Rühren aufgeheizt. Dann wurden 53 g (0,28 Mol) p-Toluolsulfonsäure hinzugegeben. Anschließend wurde das Reaktionsgemisch weiter auf ca. 115 °C aufgeheizt, wobei gleichzeitig ein Vakuum von ca. 20 mbar angelegt wurde. Unter diesen Bedingungen wurde während etwa 4 Stunden lang gerührt und dabei das Reaktionswasser im Vakuum abgezogen. Die resultierende gelblich trübe Reaktionslösung wurde auf 90 °C abgekühlt und bei Normaldruck mit 35 g (0,87 Mol) Magnesiumoxid versetzt. Daraufhin wurde 30 Minuten nachgerührt. In der Reaktionsmischung wurde ein pH-Wert von ca. 10, gemessen. Danach wurde bei einem Vakuum von 0,5 bis 1 mbar der Alkoholüberschuß abdestilliert, wobei man die Sumpftemperatur im Laufe von 3 Stunden auf 170 °C erhöhte. Während der insgesamt 3 Stunden lang dauernden Destillation, die im Reaktionskessel durchgeführt wurde, wurden ca. 20 kg Fettalkohol abdestilliert. Der Destillationsrückstand stellte eine orangerote klare Schmelze dar, die auf ca. 105 °C abgekühlt und dann mit entsalztem Wasser von 70 °C zu einer ca. 50%igen Paste vermischt wurde. Dann wurden 100 ml 50%ige Natronlauge als 1 Portion und 200 ml 35%iges Wasserstoffperoxid in 5 Portionen während 2 1/2 Stunden hinzugegeben. Danach wurde das Gemisch weitere 5 Stunden bei 80 °C gerührt. Als Reaktionsendprodukt wurden 18,9 kg einer hellgelben transparenten Paste (49,1 % Wasser, pH-Wert 9 bis 10) erhalten.

Produktkenndaten: Hydroxylzahl: 694; restlicher Fettalkohol: 1,8 %; Monoglucosid: 51 Gew.-%; Diglucosid: 16 Gew.-%; Triglucosid: 6 Gew.-%; Tetraglucosid: 4 Gew.-%; Pentaglucosid: 2 Gew.-%; Hexaglucosid unter 1 Gew.-%; Polyglucose: ca. 17 Gew.-%; Salze unter 2 Gew.-%. Klettzahlen: 20/20.

Nach 1/2jähriger Lagerung des Produkts waren die Farbzahlen und die Zusammensetzung (gaschromatographisch bestimmt) unverändert.

Zum Vergleich wurde die Farbstabilität eines nach den Lehren des Standes der Technik hergestellten Produkts bestimmt. Dieses Produkt wurde nach Beispiel 6 von US 3,839,318, Mansfield, mit einer Dodecanol/Tetradecanol-Mischung hergestellt, allerdings wurde zum Neutralisieren des saurem Katalysators (Schwefelsäure) nicht die wäßrige Natronlauge des Beispiels 6, sondern gemäß EP 132 046 B1 Natriumethylat als Base benutzt und der pH-Wert auf 7,0 eingestellt. Das so erhaltene Produkt hatte die Klettzahl 45 bzw. 125 nach dem Behandeln mit Alkali gemäß Farbstabilitätstest. Nach Überführen in eine 60%ige Paste und Bleichen mit H₂O₂ wurden die Klettzahlen 25 (direkt nach der Bleiche, pH-Wert kleiner als 7) bzw. 110 (nach Farbstabilitätstest) gemessen. Bei einer Wiederholung des Versuchs wurde der Destillationsrückstand lediglich auf 130 °C abgekühlt. Das resultierende Verfahrensprodukt hatte die gleichen Kenndaten.

### Beispiel 3:

### Dieses Beispiel beschreibt die Herstellung von C₁₂/C₁₄-Alkylglucosid in einem großtechnischen Ansatz

Von der Gesamtmenge von 1860 kg C₁₂/C₁₄-Fettalkohol (Verteilung: Dodecanol ca. 75 Gew.-%, Tetradecanol ca. 25 Gew.-%) wurde die Hälfte in einem 2,5 m³-Reaktor zusammen mit 300 kg wasserfreier Glucose (Puridex) zu einer Suspension verarbeitet. Die Feindispergierung der Suspension erfolgte mit Hilfe eines Stator/Rotor-Mischers, wobei sich die Suspension auf 75 °C erwärmte. In einem zweiten Reaktor (3,2 m³) mit Destillationskolonne und externem Flüssigkeitskreislauf, der aus einer Pumpe und einem Wärmeaustauscher bestand, wurde der restliche Fettalkohol zusammen mit 3,9 kg p-Toluolsulfonsäure vorgelegt und auf 115 °C erhitzt. Der Reaktor wurde dann auf einen Druck von 20 bis 30 mbar evakuiert. Dann wurde während 1 Stunde die Glucose/Fettalkohol-Suspension kontinuierlich hinzugegeben. Während dieser Zeit und einer Nachreaktionszeit von 2 Stunden wurden insgesamt 30 kg Wasser abdestilliert. Die zur Entfernung des Wassers und zur Aufrechterhaltung der Reaktionstemperatur notwendige Wärmeenergie wurde über den externen Flüssigkeitskreislauf in das Reaktionsgemisch eingebracht. Das Reaktionswasser wurde in einer gekühlten Vorlage aufgefangen und gemessen. Nach beendeter Reaktion wurde auf 90 °C abgekühlt. Dann wurden zur Neutralisation des sauren Katalysators 2,9 kg Magnesiumethylat in fester Form über den externen Flüssigkeitskreislauf eingesaugt. Danach wurde Normaldruck eingestellt.

Anschließend wurde das Reaktionsgemisch in einen Dünnschichtverdampfer Typ Sambay (0,75 qm Verdampferfläche, 8 mbar, ca. 170°C) geleitet und der überschüssige Fettalkohol bis auf einen Abreicherungswert von ca. 32 % abgetrennt. Das bei 135 °C gehaltene Produkt war niedrig viskos und konnte leicht in einen Kurzwegverdampfer mit Rollenwischer vom Typ KD 75, Fa. Leybold, übergeführt werden. Der Kurzwegverdampfer wurde unter den folgenden Bedingungen betrieben: Verdampferfläche 0,75 qm; Arbeitsdruck 0,075 mbar, im Verdampfer gemessen; Beheizungstemperatur 170 °C; Sumpfablauftemperatur 162 °C. Alternativ wurde in einem zweiten Ansatz ein Dünnschichtverdampfer auch in der zweiten Abreicherungsstufe verwendet. Das Produkt wurde chargenweise (90 kg) in geschmolzenem Zustand bei 150 °C in einem Druckkessel mit ca. 88 kg Wasser von Raumtemperatur versetzt, um so eine ca. 50%ige Paste herzustellen. Separat wurden 1,3 kg einer 35%igen H₂O₂-Lösung und 0,9 kg einer 50%igen NaOH-Lösung zudosiert. Nach 3stündigem Rühren bei 90 °C wurde auf 50 °C abgekühlt.

Produktkenndaten: pH-Wert 9,5; Klettzahl 23 (nach der Bleiche) bzw. 26 nach dem Behandeln mit Alkali und in der Wärme nach dem Farbstabilitätstest. Zusammensetzung des Produkts (wasserfrei): Hydroxylzahl 650; restlicher Fettalkohol 3 Gew.-%; Alkylmonoglucosid 62,8 Gew.-%; Alkyldiglucosid 15,4 Gew.-%; Alkyltriglucosid 5,8 Gew.-%; Alklyltetraglucosid 2,5 Gew.-%; Alkylpentaglucosid 1,1 Gew.-%; Alkylhexaglucosid 0,2 Gew.-%; Polyglucose 6 Gew.-%; Glucose weniger als 1 Gew.-%; Salze weniger als 2 Gew.-%.

## Patentansprüche

1. Verfahren zur direkten Herstellung von Alkylglykosiden durch Acetalisierungsreaktion von höheren aliphatischen primären Alkoholen mit Glykosen, insbesondere mit Glucose, in Gegenwart eines sauren Katalysators, rascher Entfernung des Reaktionswassers, Neutralisation des Katalysators mit einer Base, Abdestillation des Alkoholüberschusses und Überführen des Reaktionsproduktes in eine wäßrige Paste und Bleichen dieser Paste, wobei der aliphatische Alkohol im molaren Überschuß zur Glykose eingesetzt wird, und wobei die Bildung und Abführung des Reaktionswassers unter Vakuum erfolgt und Reaktionstemperaturen über 80 °C angewendet werden, dadurch gekennzeichnet, daß man:
a) Mischungen aus aliphatischem primärem Alkohol, Glykose und saurem Katalysator bei erhöhter Temperatur erzeugt und zur Reaktion bringt, wobei man entweder
(i) eine Teilmenge des Alkohols mit dem Katalysator vorlegt, die Mischung erwärmt, und dann eine erwärmte Suspension der Glykose in der restlichen Alkoholmenge portionsweise oder kontinuierlich zu der Alkohol/Katalysatormischung zudosiert und dabei im Vakuum das entstehende Reaktionswasser abdestilliert, oder
(ii) eine Mischung des gesamten Alkohols und der Glykose vorlegt, erwärmt und den sauren Katalysator zur erwärmten Mischung hinzugibt, dann ein Vakuum anlegt und weiter bis zum Einsetzen der Reaktion erwärmt und das entstehende Reaktionswasser abdestilliert,
b) dabei die Ansatzverhältnisse so wählt, daß das Molverhältnis Glykose zu aliphatischem Alkohol bei 1 : 2 bis 1 : 10, vorzugsweise 1 : 3 bis 1 : 6 liegt,
c) das Reaktionsgemisch bei dieser Temperatur und diesem Unterdruck so lange hält, vorzugsweise unter Durchmischung, bis das Reaktionswasser vollständig entfernt ist,
d) anschließend das Reaktionsgemisch auf ca. 90 ° abkühlt, dann eine organische oder anorganische basische Alkali-, Erdalkali- oder Aluminium- bzw. Alkali/Aluminiumverbindung in solchen Mengen hinzufügt, daß sich über die Neutralisation des sauren Katalysators hinaus ein pH-Wert von wenigstens 8, vorzugsweise 8 bis 10 einstellt, und vorzugsweise erst danach Normaldruck herstellt,
e) daß man vorzugsweise ohne vorherige Filtration den überschüssigen Alkohol aus dem alkalischen Gemisch im Vakuum auf an sich übliche, das Reaktionsprodukt schonende Weise auf einen Wert von unterhalb 5 Gew.-% des Reaktionsproduktes abdestilliert, und
f) daß man anschließend auf ca. 105 °C bis 130 °C abkühlt und durch Hinzugabe von Wasser eine 30- bis 70prozentige Paste erzeugt, die man durch vorzugsweise portionsweises Hinzufügen von Aktivsauerstoffverbindungen, vorzugsweise Wasserstoffperoxid, ca. 0,1 bis 5 Stunden bei ca. 80 °C rührt, wobei man gegebenenfalls durch Hinzufügen von Alkali, vorzugsweise Natronlauge, dafür sorgt, daß der pH-Wert während dieses Bleichprozesses bei 8 bis 10 bleibt.

2. Verfahren nach Anspruch 1, dadurch gekennzeichnet, daß man als höhere aliphatische primäre Alkohole solche mit 8 bis 20 Kohlenstoffatomen, vorzugsweise 12 bis 18 Kohlenstoffatomen einsetzt.

3. Verfahren nach den Ansprüchen 1 oder 2, dadurch gekennzeichnet, daß man als Teilmenge etwa 30 bis 70 Gew.-%. des Alkohols zusammen mit dem Katalysator vorlegt, die Mischung auf etwa 100 bis 120 °C erwärmt, und dann die Glykose in erwärmter Suspension in der restlichen Alkoholmenge, vorzugsweise kontinuierlich unter Vakuum, hinzugibt und das entstehende Reaktionswasser abdestilliert.

4. Verfahren nach den Ansprüchen 1 oder 2, dadurch gekennzeichnet, daß man eine Mischung der gesamten Alkoholmenge und der Glykose voriegt, erwärmt, dann den sauren Katalysator zu der erwärmten Mischung hinzugibt, ein leichtes Vakuum anlegt und weiter bis auf ca. 100 bis 120 °C erwärmt und das entstehende Reaktionswasser abdestilliert.

5. Verfahren nach einem der Ansprüche 1 bis 4, dadurch gekennzeichnet, daß man die Glykose/Alkohol-Suspension einer Feindispergierung unterzieht.

6. Verfahren nach einem der Ansprüche 1 bis 5, dadurch gekennzeichnet, daß man das Vakuum so einstellt, daß der Siedepunkt des Alkohols um wenigstens 30 °C gesenkt wird, vorzugsweise daß man ein Vakuum von 10 bis 50 mbar einstellt.

7. Verfahren nach einem der Ansprüche 1 bis 6, dadurch gekennzeichnet, daß man als höhere aliphatische primäre Alkohole gesättigte C₁₂-C₁₈-Alkohole, die vorzugsweise geradkettig sind, einsetzt.

8. Verfahren nach einem der Ansprüche 1 bis 7, dadurch gekennzeichnet, daß man als saure Katalysatoren solche Verbindungen und in solchen Mengen verwendet, daß deren resultierende Alkali-, Erdalkali- bzw. Aluminium-Salze im Produkt verbleiben können, vorzugsweise eine Säure aus der Gruppe, bestehend aus Schwefelsäure, Phosphorsäure oder aliphatische und/oder aromatische Sulfonsäuren, vorzugsweise Paratoluolsulfonsäure, in einer Menge von vorzugsweise 0,005 bis 0,02 Mol pro Mol der eingesetzten Glykose.

9. Verfahren nach einem der Ansprüche 1 bis 8, dadurch gekennzeichnet, daß man als basische Substanzen für die Neutralisation des sauren Katalysators und darüber hinaus für die Einstellung eines basischen pH-Werts anorganische, feinpulvrisierte Verbindungen aus der Gruppe, bestehend aus Calciumhydroxid, Calciumoxid, Magnesiumhydroxid, Magnesiumoxid, die Zeolithe NaA oder NaX, vorzugsweise in Kombination mit Calciumhydroxid, und als organische Verbindungen die Alkoholate von niedrig siedenden Alkoholen, vorzugsweise C₁-C₄-Alkoholen, in Form der Alkalimetall- und/oder Erdalkalimetall-Verbindungen, einsetzt.

10. Verfahren nach einem der Ansprüche 1 bis 9, dadurch gekennzeichnet, daß man als anorganische basische Verbindung Magnesiumoxid und als organische basische Verbindung ein Magnesiumalkoholat, insbesondere Magnesiumethylat einsetzt.

11. Verfahren nach einem der Ansprüche 1 bis 10, dadurch gekennzeichnet, daß man nach der Neutralisation die basische Reaktionsmischung unter einem Vakuum, das zur Abdestillation des Alkoholüberschusses erforderlich ist, bis auf eine Sumpftemperatur von 160 bis 180 °C, insbesondere von 150 bis 170 °C erhitzt, wobei man auch bei Ansätzen mit kürzerkettigen Alkoholen mit entsprechend niederen Siedepunkten diese hohe Sumpftemperatur einstellt.

12. Verfahren nach Anspruch 1, gekennzeichnet durch die Anwendung der folgenden kumulativen Verfahrensschritte:
1. Die Glykose, insbesondere die Glucose, wird im Alkohol mit hochtourigen Rührern oder mit anderen hochwirksamen technischen Mischvorrichtungen feindispergiert.
2. Die zur Neutralisation des Säurekatalysators, vorzugsweise einer Sulfonsäure, verwendete Base besteht ganz oder überwiegend aus Magnesiumoxid.
3. Die Basenmenge wird so berechnet, daß über die eigentliche Neutralisation hinaus eine basisch reagierende Mischung, vorzugsweise von pH 8 bis 10 erhalten wird.
4. Das Reaktionsgemisch wird nach der Neutralisation nicht filtriert.
5. Beim Abdestillieren des überschüssigen Alkohols im Vakuum wird schließlich auf eine Sumpftemperatur von 150 bis 180 °C erhitzt bzw. die Beheizungstemperatur im Verdampfergerät der zweiten Stufe auf etwa 170 °C bis 180 °C gebracht.

13. Alkylglykosid als Erzeugnis nach einem der Ansprüche 1 bis 12, dadurch gekennzeichnet, daß dessen Oligomerisierungsgrad bei 1 bis 5, vorzugsweise 1 bis 1,5, liegt und insbesondere so eingestellt wird, daß die Menge an Alkylmonoglykosid, bezogen auf die Gesamtmenge aus Alkylmonoglykosid und Alkyloligoglykosid, deutlich über 70 Gew.-% liegt.

14. Erzeugnis nach Anspruch 13, dadurch gekennzeichnet, daß die Restalkoholmenge, bezogen auf das wasserfreie Produkt, auf 0,2 bis 5, insbesondere 0,5 bis 2,5 Gew.-% eingestellt ist.

15. Erzeugnis nach den Ansprüchen 13 und 14, dadurch gekennzeichnet, daß es in Form einer wäßrigen Paste mit 30 bis 60 Gew.-% Wasseranteil vorliegt, welche die aus der Neutralisation des Katalysators und dem Bleichvorgang stammenden Salze enthält.

16. Erzeugnis in Form einer wäßrigen Paste nach einem der Ansprüche 13 bis 15, dadurch gekennzeichnet, daß es einen die Lagerungsbeständigkeit verbessernden antimikrobiellen Zusatz in Mengen von 0,1 bis 0,2 Gew.-% enthält.

## Claims

1. A process for the direct production of alkyl glycosides by acetalization of higher aliphatic primary alcohols with glycoses, particularly glucose, in the presence of an acidic catalyst, rapid removal of the water of reaction, neutralization of the catalyst with a base, removal of the alcohol excess by distillation and conversion of the reaction product into an aqueous paste and bleaching of this paste, the aliphatic alcohol being used in a molar excess to the glycose and the formation and removal of the water of reaction taking place in vacuo and reaction temperatures above 80°C being applied, characterized in that
a) mixtures of aliphatic primary alcohol, glycose and acidic catalyst are prepared and reacted at elevated temperature, either
(i) part of the alcohol being initially introduced with the catalyst, the mixture being heated and a heated suspension of the glycose in the remaining quantity of alcohol being added continuously or in portions to the alcohol/catalyst mixture and the water of reaction formed being distilled off in vacuo, or
(ii) a mixture of the entire alcohol and the glycose being initially introduced, heated and the acidic catalyst being added to the heated mixture, a vacuum subsequently being applied and the mixture being further heated until the reaction begins and the water of reaction formed being distilled off,
b) the mixing ratios are selected so that the molar ratio of glycose to aliphatic alcohol is from 1:2 to 1:10 and preferably from 1:3 to 1:6,
c) the reaction mixture is kept at that temperature and under that reduced pressure, preferably while mixing, until the water of reaction has been completely removed,
d) the reaction mixture is subsequently cooled to approximately 90°C, after which an organic or inorganic basic alkali metal, alkaline-earth metal or aluminium or alkali metal/aluminium compound is added in such quantities that, over and above the neutralization of the acidic catalyst, a pH value of at least 8 and preferably in the range from 8 to 10 is established and normal pressure is preferably only established thereafter,
e) the excess alcohol is distilled off from the alkaline mixture in vacuo, preferably without preliminary filtration, to a value below 5% by weight of the reaction product by any of the methods known per se which do not damage the reaction product and
f) the mixture is subsequently cooled to approximately 105 to 130°C and a 30 to 70% paste is produced by addition of water and is stirred for about 0.1 to 5 hours at approximately 80°C by the addition, preferably in portions, of active oxygen compounds, preferably hydrogen peroxide, measures optionally being taken by addition of alkali, preferably sodium hydroxide, to ensure that the pH value remains at 8 to 10 during this bleaching process.

2. A process as claimed in claim 1, characterized in that the higher aliphatic primary alcohols used are those containing 8 to 20 carbon atoms and preferably 12 to 18 carbon atoms.

3. A process as claimed in claim 1 or 2, characterized in that part of the alcohol, i.e. about 30 to 70% by weight, is introduced together with the catalyst, the mixture is heated to around 100 to 120°C and the glycose is then added, preferably continuously in vacuo, in the form of a heated suspension in the remaining alcohol and the water of reaction formed is distilled off.

4. A process as claimed in claim 1 or 2, characterized in that a mixture of the entire quantity of alcohol and the glycose is introduced and heated, the acidic catalyst is added to the heated mixture, a slight vacuum is applied and the mixture is further heated to approximately 100 to 120°C and the water of reaction is distilled off.

5. A process as claimed in any of claims 1 to 4, characterized in that the glycose/alcohol suspension is subjected to fine dispersion.

6. A process as claimed in any of claims 1 to 5, characterized in that the vacuum is adjusted so that the boiling point of the alcohol is lowered by at least 30°C, a vacuum of 10 to 50 mbar preferably being applied.

7. A process as claimed in any of claims 1 to 6, characterized in that saturated, preferably linear, C₁₂₋₁₈ alcohols are used as the higher aliphatic primary alcohols.

8. A process as claimed in any of claims 1 to 7, characterized in that such compounds are used as the acidic catalysts in such quantities that their resulting alkali metal, alkaline earth metal or aluminium salts can remain in the product, preferably an acid from the group consisting of sulfuric acid, phosphoric acid or aliphatic and/or aromatic sulfonic acids, preferably p-toluenesulfonic acid in a quantity of preferably from 0.005 to 0.02 mol per mol of the glycose used.

9. A process as claimed in any of claims 1 to 8, characterized in that inorganic, finely powdered compounds from the group consisting of calcium hydroxide, calcium oxide, magnesium hydroxide, magnesium oxide, the zeolites NaA or NaX, preferably in combination with calcium hydroxide, are used as the bases for neutralizing the acidic catalyst and, in addition, for establishing a basic pH value while the alcoholates of low-boiling alcohols, preferably C₁₋₄ alcohols, in the form of the alkali metal and/or alkaline earth metal compounds are used as the organic compounds.

10. A process as claimed in any of claims 1 to 9, characterized in that magnesium oxide is used as the inorganic basic compound and a magnesium alcoholate, more especially magnesium ethylate, is used as the organic basic compound.

11. A process as claimed in any of claims 1 to 10, characterized in that, after the neutralization step, the basic reaction mixture is heated under a vacuum which is necessary for distilling off the alcohol excess, to a sump temperature of from 160 to 180°C and more especially from 160 to 170°C, this high sump temperature being adjusted even in the case of mixtures containing relatively short-chain alcohols with correspondingly lower boiling points.

12. A process as claimed in claim 1, characterized by application of the following cumulative process steps:
1. the glycose, particularly the glucose, is finely dispersed in the alcohol by high-speed stirrers or by other high-performance industrial mixers;
2. the base used to neutralize the acid catalyst, preferably a sulfonic acid, consists completely or predominantly of magnesium oxide;
3. the quantity of base is calculated so that, over and above the actual neutralization, a basically reacting mixture, preferably of pH 8 to 10, is obtained;
4. the reaction mixture is not filtered after the neutralization step;
5. finally, during removal of the excess alcohol by distillation in vacuo, the reaction mixture is heated to a sump temperature of 160 to 180°C or the heating temperature in the evaporator of the second stage is brought to about 170 to 180°C.

13. Alkyl glycoside as the product of the process claimed in any of claims 1 to 12, characterized in that its degree of oligomerization is in the range from 1 to 5 and preferably from 1 to 1.5 and, in particular, is adjusted so that the quantity of alkyl monoglycoside, based on the total quantity of alkyl monoglycoside and alkyl oligoglycoside, distinctly exceeds 70% by weight.

14. The product claimed in claim 13, characterized in that the quantity of residual alcohol, based on the anhydrous product, is adjusted to between 0.2 and 5% by weight and more especially to between 0.5 and 2.5% by weight.

15. The product claimed in claims 13 and 14, characterized in that it is present in the form of an aqueous paste containing 30 to 60% by weight water which contains the salts emanating from neutralization of the catalyst and from the bleaching process.

16. The product in the form of an aqueous paste claimed in any of claims 13 to 15, characterized in that it contains an antimicrobial additive improving stability in storage in quantities of from 0.1 to 0.2% by weight.

## Revendications

1. Procédé de fabrication directe d'alkylglycosides par réaction d'acétalisation d'alcools primaires aliphatiques supérieurs avec des oses, en particulier du glucose, en présence d'un catalyseur acide, élimination rapide de l'eau de réaction, neutralisation du catalyseur par une base, élimination par distillation de l'alcool en excès, transformation du produit de réaction en une pâte aqueuse et décoloration de cette pâte, l'alcool aliphatique étant mis en oeuvre en excès molaire par rapport à l'ose, la formation et l'évacuation de l'eau de réaction se déroulant sous vide et des températures de réaction supérieures à 80 °C étant appliquées, caractérisé en ce que l'on:
a) confectionne et met en réaction les mélanges d'alcool primaire aliphatique, d'ose et de catalyseur acide à température accrue, soit
(i) en préparant un mélange d'une quantité partielle de l'alcool avec le catalyseur, en chauffant ledit mélange et en ajoutant ensuite progressivement une suspension chauffée de l'ose dans la quantité restante de l'alcool, par portions ou continuellement, au mélange d'alcool et de catalyseur et en éliminant l'eau de réaction formée par distillation sous vide, soit
(ii) en préparant et en chauffant un mélange de la totalité de l'alcool et de l'ose, et en ajoutant le catalyseur acide au mélange chauffé, puis en appliquant un vide, en continuant à chauffer jusqu'à l'amorçage de la réaction et en éliminant par distillation l'eau de réaction formée,
b) sélectionne les rapports de mélange, de manière telle que le rapport molaire entre l'ose et l'alcool aliphatique se situe dans l'intervalle de 1:2 à 1:10, de préférence de 1:3 à 1:6,
c) maintient le mélange réactionnel à cette température et sous cette dépression jusqu'à ce que l'eau de réaction soit totalement éliminée, de préférence sous mélangeage intime,
d) refroidit ensuite le mélange réactionnel à environ 90 °C, puis ajoute un composé basique, organique ou inorganique de métal alcalin, de métal alcalino-terreux ou d'aluminium ou de métal alcalin et d'aluminium, en quantités telles qu'un pH d'au moins 8, de préférence de 8 à 10, s'instaure pendant toute la neutralisation du catalyseur acide et que la pression normale soit rétablie de préférence seulement ensuite,
e) élimine par distillation l'alcool en excès du mélange alcalin sous vide, de préférence sans filtration préalable, d'une manière usuelle en soi ménageant le produit de réaction, jusqu'à une teneur inférieure à 5 % en poids du produit réactionnel et
f) ramène ensuite la température à environ 105 à 130 °C et confectionne une pâte à 30 à 70 pour cent par adjonction d'eau, que l'on remue sous adjonction, de préférence par portions, de composés d'oxygène actif, de préférence d'eau oxygénée, pendant environ 0,1 à 5 heures à environ 80 °C, en veillant à ce que le pH reste dans l'intervalle de 8 à 10 pendant ce processus de décoloration, le cas échéant par adjonction d'alcalis, de préférence de soude caustique.

2. Procédé selon la revendication 1, caractérisé en ce que l'on utilise comme alcools primaires aliphatiques supérieurs ceux comportant 8 à 20 atomes de carbone, de préférence 12 à 18 atomes de carbone.

3. Procédé selon la revendication 1 ou 2, caractérisé en ce que l'on prépare comme quantité partielle environ 30 à 70 % en poids de l'alcool, conjointement avec le catalyseur, en ce que l'on porte la température du mélange à environ 100 à 120 °C, et en ce que l'on ajoute ensuite l'ose en suspension chauffée dans la quantité restante d'alcool, de préférence continuellement sous vide, et en ce que l'on élimine l'eau de réaction formée par distillation.

4. Procédé selon la revendication 1 ou 2, caractérisé en ce que l'on prépare un mélange de la quantité d'alcool totale et de l'ose, en ce que l'on chauffe et en ce que l'on ajoute ensuite le catalyseur acide au mélange chauffé, applique un léger vide, continue à chauffer jusqu'à environ 100 à 120 °C et élimine par distillation l'eau de réaction formée.

5. Procédé selon l'une des revendications 1 à 4, caractérisé en ce que l'on soumet la suspension d'ose/alcool à une dispersion fine.

6. Procédé selon l'une des revendications 1 à 5, caractérisé en ce que l'on instaure le vide de manière telle que le point d'ébullition de l'alcool est abaissé d'au moins 30 °C, de préférence en ce que l'on instaure un vide de 10 à 50 mbars.

7. Procédé selon l'une des revendications 1 à 6, caractérisé en ce que l'on met en oeuvre comme alcools primaires aliphatiques supérieurs des alcools saturés de C₁₂ à C₁₈, qui sont de préférence à chaîne droite.

8. Procédé selon l'une des revendications 1 à 7, caractérisé en ce que l'on utilise comme catalyseurs acides des composés tels et en quantités telles, que leurs sels de métaux alcalins, de métaux alcalino-terreux ou d'aluminium résultants peuvent rester dans le produit, de préférence un acide faisant partie du groupe constitué de l'acide sulfurique, de l'acide phosphorique ou des acides sulfoniques aliphatiques et/ou aromatiques, de préférence de l'acide paratoluènesulfonique, dans une proportion allant de préférence de 0,005 à 0,02 mole par mole d'ose mis en oeuvre.

9. Procédé selon l'une des revendications 1 à 8, caractérisé en ce que l'on met en oeuvre comme substances basiques pour la neutralisation du catalyseur acide et en outre pour l'instauration d'un pH basique, des composés inorganiques finement pulvérisés faisant partie du groupe constitué de l'hydroxyde de calcium, de l'oxyde de calcium, de l'hydroxyde de magnésium, de l'oxyde de magnésium, des zéolithes NaA ou NaX, de préférence en association avec l'hydroxyde de calcium, et comme composés organiques, des alcoolates d'alcools à point d'ébullition bas, de préférence d'alcools de C₁ à C₄, sous forme de composés de métaux alcalins et/ou de métaux alcalino-terreux.

10. Procédé selon l'une des revendications 1 à 9, caractérisé en ce que l'on met en oeuvre comme composé basique inorganique, de l'oxyde de magnésium et comme composé basique organique, un alcoolate de magnésium, en particulier du méthylate de magnésium.

11. Procédé selon l'une des revendications 1 à 10, caractérisé en ce qu'après la neutralisation, on chauffe le mélange réactionnel de base, sous un vide qui est indispensable à l'élimination par distillation de l'alcool en excès, jusqu'à une température de résidu (de distillation) de 160 à 180 °C, en particulier de 160 à 170 °C, en instaurant cette température élevée de résidu également pour les préparations avec des alcools à chaîne plus courte à point d'ébullition bas approprié.

12. Procédé selon la revendication 1, caractérisé par la mise en oeuvre des étapes de procédé cumulatives suivantes:
1. L'ose, en particulier le glucose, est finement dispersé dans l'alcool au moyen de mélangeurs à vitesse élevée ou d'autres dispositifs techniques de mélangeage à efficacité élevée.
2. La base utilisée pour la neutralisation du catalyseur acide, de préférence un acide sulfonique, est constituée en totalité ou en majeure partie d'oxyde de magnésium.
3. La quantité de base est calculée de manière telle qu'un mélange à réaction basique, de préférence avec un pH de 8 à 10, est obtenu pendant toute la neutralisation proprement dite.
4. Le mélange réactionnel n'est pas filtré après la neutralisation.
5. Pour l'élimination par distillation de l'alcool en excès dans le vide, on chauffe finalement à une température de résidu de 160 à 180 °C ou la température de chauffage dans l'appareil d'évaporation de la deuxième étape est portée à environ 170 à 180 °C.

13. Alkylglycoside comme produit obtenu selon l'une des revendications 1 à 12, caractérisé en ce que son degré d'oligomérisation est de 1 à 5, de préférence de 1 à 1,5 et est en particulier ajusté de manière telle que la quantité d'alkylmonoglycoside, par rapport à la quantité totale d'alkylmonoglycoside et d'alkyloligoglycoside, est nettement supérieure à 70 % en poids.

14. Produit selon la revendication 13, caractérisé en ce que la quantité résiduelle d'alcool, par rapport au produit anhydre, est ajustée à 0,2 à 5, en particulier à 0,5 à 2,5 % en poids.

15. Produit selon les revendications 13 et 14, caractérisé en ce qu'il est présent sous la forme d'une pâte aqueuse avec une proportion d'eau de 30 à 60 % en poids, qui renferme des sels provenant de la neutralisation du catalyseur et du processus de décoloration.

16. Produit sous la forme d'une pâte aqueuse selon l'une des revendications 13 à 15, caractérisé en ce qu'il renferme un additif antimicrobien améliorant la stabilité au stockage, en proportions de 0,1 à 0,2 % en poids.
